# EUROPEAN PATENT APPLICATION

(11) **EP 1 616 879 A2**
(43) Date of publication of application: **18.01.2006**
(21) Application number: 05019550.2
(22) Date of filing: 08.10.2000
(51) Int. Cl.: C07K 14/47

(54) **Human cub-domain-containing protein and gene encoding the same**

(30) Priority: 19.10.1999 US 160285 P; 18.02.2000 US 183583 P
(62) Divisional of application: 00972258.8
(71) Applicant: Lexicon Genetics Incorporated, The Woodlands, TX 77381-1160 (US)
(72) Inventor: Donoho, Gregory, IN 46259 (US); Turner, Alexander C. Jr., The Woodlands TX 77381 (US); Nehls, Michael, 82131 Stockdorf (DE); Friedrich, Glenn, Houston TX 77004 (US); Zambrowicz, Brian, The Woodlands TX 77382 (US); Sands, Arthur T., The Woodlands TX 77382 (US)
(74) Representative: Silveston, Judith

(57) **Abstract**

A human protein containing a cub-domain and its corresponding gene have been identified. The related polypeptidic and polynucleotidic sequences here disclosed can have therapeutic, diagnostic and pharmacogenomic applications.

Sequence Listing

<110> Donoho, Gregory
      Turner, C. Alexander Jnr.
      Nehls, Michael
      Friedrich, Glenn
      Zambrowicz, Brian
      Sands, Arthur T.

<120> Novel Human Proteins and Polynucleotides
  Encoding the Same

<130> 8204(1)/JSvn/TJH

<150> US 60/160,285
<151> 1999-10-19

<150> US 60/183,583
<151> 2000-02-18

<160> 7

<170> FastSEQ for Windows Version 4.0

<210> 1
<211> 1464
<212> DNA
<213> homo sapiens

<400> 1
atgacatcta agaattatcc cgggacctac cccaatcaca ctgtttgcga aaagacaatt 60
acagtaccaa aggggaaaag actgattctg aggttgggag atttggatat cgaatcccag 120
acctgtgctt ctgactatct tctcttcacc agctcttcag atcaatatgg tccatactgt 180
ggaagtatga ctgttcccaa agaactcttg ttgaacacaa gtgaagtaac cgtccgcttt 240
gagagtggat cccacatttc tggccggggt tttttgctga cctatgcgag cagcgaccat 300
ccagatttaa taacatgttt ggaacgagct agccattatt tgaagacaga atacagcaaa 360
ttctgcccag ctggttgtag agacgtagca ggagacattt ctgggaatat ggtagatgga 420
tatagagata cctctttatt gtgcaaagct gccatccatg caggaataat tgctgatgaa 480
ctaggtggcc agatcagtgt gcttcagcgc aaagggatca gtcgatatga agggattctg 540
gccaatggtg ttctttcgag ggatggttcc ctgtcagaca agcgatttct gtttacctcc 600
aatggttgca gcagatcctt gagttttgaa cctgacgggc aaatcagagc ttcttcctca 660
tggcagtcgg tcaatgagag tggagaccaa gttcactggt ctcctggcca agcccgactt 720
caggaccaag gcccatcatg ggcttcgggc gacagtagca acaaccacaa accacgagag 780
tggctggaga tcgatttggg ggagaaaaag aaaataacag gaattaggac cacaggatct 840
acacagtcga acttcaactt ttatgttaag agttttgtga tgaacttcaa aaacaataat 900
tctaagtgga agacctataa aggaattgtg aataatgaag aaaaggtgtt tcagggtaac 960
tctaactttc gggacccagt gcaaaacaat ttcatccctc ccatcgtggc cagatatgtg 1020
cgggttgtcc cccagacatg gcaccagagg atagccttga aggtggagct cattggttgc 1080
cagattacac aaggtaatga ttcattggtg tggcgcaaga caagtcaaag caccagtgtt 1140
tcaactaaga aagaagatga gacaatcaca aggcccatcc cctcggaaga aacatccaca 1200
ggaataaaca ttacaacggt ggctattcca ttggtgctcc ttgttgtcct ggtgtttgct 1260
ggaatgggga tctttgcagc ctttagaaag aagaagaaga aaggaagtcc gtatggatca 1320
gcggaggctc agaaaacaga ctgttggaag cagattaaat atccctttgc cagacatcag 1380
tcagctgagt ttaccatcag ctatgataat gagaaggaga tgacacaaaa gttagatctc 1440
atcacaagtg atatggcagg ttaa 1464

<210> 2
<211> 487
<212> PRT
<213> homo sapiens

<400> 2
Met Thr Ser Lys Asn Tyr Pro Gly Thr Tyr Pro Asn His Thr Val Cys
 1               5                  10                  15
Glu Lys Thr Ile Thr Val Pro Lys Gly Lys Arg Leu Ile Leu Arg Leu
            20                  25                  30
Gly Asp Leu Asp Ile Glu Ser Gln Thr Cys Ala Ser Asp Tyr Leu Leu
        35                  40                  45
Phe Thr Ser Ser Ser Asp Gln Tyr Gly Pro Tyr Cys Gly Ser Met Thr

50                  55                  60
Val Pro Lys Glu Leu Leu Leu Asn Thr Ser Glu Val Thr Val Arg Phe
65                  70                  75                  80
Glu Ser Gly Ser His Ile Ser Gly Arg Gly Phe Leu Leu Thr Tyr Ala
                85                  90                  95
Ser Ser Asp His Pro Asp Leu Ile Thr Cys Leu Glu Arg Ala Ser His
            100                 105                 110
Tyr Leu Lys Thr Glu Tyr Ser Lys Phe Cys Pro Ala Gly Cys Arg Asp
        115                 120                 125
Val Ala Gly Asp Ile Ser Gly Asn Met Val Asp Gly Tyr Arg Asp Thr
    130                 135                 140
Ser Leu Leu Cys Lys Ala Ala Ile His Ala Gly Ile Ile Ala Asp Glu
145                 150                 155                 160
Leu Gly Gly Gln Ile Ser Val Leu Gln Arg Lys Gly Ile Ser Arg Tyr
                165                 170                 175
Glu Gly Ile Leu Ala Asn Gly Val Leu Ser Arg Asp Gly Ser Leu Ser
            180                 185                 190
Asp Lys Arg Phe Leu Phe Thr Ser Asn Gly Cys Ser Arg Ser Leu Ser
        195                 200                 205
Phe Glu Pro Asp Gly Gln Ile Arg Ala Ser Ser Ser Trp Gln Ser Val
    210                 215                 220
Asn Glu Ser Gly Asp Gln Val His Trp Ser Pro Gly Gln Ala Arg Leu
225                 230                 235                 240
Gln Asp Gln Gly Pro Ser Trp Ala Ser Gly Asp Ser Ser Asn Asn His
                245                 250                 255
Lys Pro Arg Glu Trp Leu Glu Ile Asp Leu Gly Glu Lys Lys Lys Ile
            260                 265                 270
Thr Gly Ile Arg Thr Thr Gly Ser Thr Gln Ser Asn Phe Asn Phe Tyr
        275                 280                 285
Val Lys Ser Phe Val Met Asn Phe Lys Asn Asn Asn Ser Lys Trp Lys
    290                 295                 300
Thr Tyr Lys Gly Ile Val Asn Asn Glu Glu Lys Val Phe Gln Gly Asn
305                 310                 315                 320
Ser Asn Phe Arg Asp Pro Val Gln Asn Asn Phe Ile Pro Pro Ile Val
                325                 330                 335
Ala Arg Tyr Val Arg Val Val Pro Gln Thr Trp His Gln Arg Ile Ala
            340                 345                 350
Leu Lys Val Glu Leu Ile Gly Cys Gln Ile Thr Gln Gly Asn Asp Ser
        355                 360                 365
Leu Val Trp Arg Lys Thr Ser Gln Ser Thr Ser Val Ser Thr Lys Lys
    370                 375                 380
Glu Asp Glu Thr Ile Thr Arg Pro Ile Pro Ser Glu Glu Thr Ser Thr
385                 390                 395                 400
Gly Ile Asn Ile Thr Thr Val Ala Ile Pro Leu Val Leu Leu Val Val
                405                 410                 415
Leu Val Phe Ala Gly Met Gly Ile Phe Ala Ala Phe Arg Lys Lys Lys
            420                 425                 430
Lys Lys Gly Ser Pro Tyr Gly Ser Ala Glu Ala Gln Lys Thr Asp Cys
        435                 440                 445
Trp Lys Gln Ile Lys Tyr Pro Phe Ala Arg His Gln Ser Ala Glu Phe
    450                 455                 460
Thr Ile Ser Tyr Asp Asn Glu Lys Glu Met Thr Gln Lys Leu Asp Leu
465                 470                 475                 480
Ile Thr Ser Asp Met Ala Gly
                485

<210> 3
<211> 1761
<212> DNA
<213> homo sapiens

<400> 3
atgggattcg gtgcggggca gcgactgcgc cccgtcccgg cgccgcgctc gtccgcagag      60
gaggcggccc ggcccgggca gctgcggctc gggatccgtc gaggggaggc cgagcttgcc     120
aagctggcgc ccagcggggt catggtgccc ggcgcccgcg gcggcggcgc actggcgcgg     180
gctgccgggc ggggcctcct ggctttgctg ctcgcggtct ccgccccgct ccggctgcag     240
gcggaggagc tgggtgatgg ctgtggacac ctagtgactt atcaggatag tggcacaatg     300
acatctaaga attatcccgg gacctacccc aatcacactg tttgcgaaaa gacaattaca     360

gtaccaaagg ggaaaagact gattctgagg ttgggagatt tggatatcga atcccagacc 420
tgtgcttctg actatcttct cttcaccagc tcttcagatc aatatggtcc atactgtgga 480
agtatgactg ttcccaaaga actcttgttg aacacaagtg aagtaaccgt ccgctttgag 540
agtggatccc acatttctgg ccggggtttt ttgctgacct atgcgagcag cgaccatcca 600
gatttaataa catgtttgga acgagctagc cattatttga agacagaata cagcaaattc 660
tgcccagctg gttgtagaga cgtagcagga gacatttctg ggaatatggt agatggatat 720
agagatacct ctttattgtg caaagctgcc atccatgcag gaataattgc tgatgaacta 780
ggtggccaga tcagtgtgct tcagcgcaaa gggatcagtc gatatgaagg gattctggcc 840
aatggtgttc tttcgaggga tggttccctg tcagacaagc gatttctgtt tacctccaat 900
ggttgcagca gatccttgag ttttgaacct gacgggcaaa tcagagcttc ttcctcatgg 960
cagtcggtca atgagagtgg agaccaagtt cactggtctc ctggccaagc ccgacttcag 1020
gaccaaggcc catcatgggc ttcgggcgac agtagcaaca accacaaacc acgagagtgg 1080
ctggagatcg atttggggga gaaaaagaaa ataacaggaa ttaggaccac aggatctaca 1140
cagtcgaact tcaactttta tgttaagagt tttgtgatga acttcaaaaa caataattct 1200
aagtggaaga cctataaagg aattgtgaat aatgaagaaa aggtgtttca gggtaactct 1260
aactttcggg acccagtgca aaacaatttc atccctccca tcgtggccag atatgtgcgg 1320
gttgtccccc agacatggca ccagaggata gccttgaagg tggagctcat tggttgccag 1380
attacacaag gtaatgattc attggtgtgg cgcaagacaa gtcaaagcac cagtgtttca 1440
actaagaaag aagatgagac aatcacaagg cccatcccct cggaagaaac atccacagga 1500
ataaacatta caacggtggc tattccattg gtgctccttg ttgtcctggt gtttgctgga 1560
atggggatct ttgcagcctt tagaaagaag aagaagaaag gaagtccgta tggatcagcg 1620
gaggctcaga aaacagactg ttggaagcag attaaatatc cctttgccag acatcagtca 1680
gctgagttta ccatcagcta tgataatgag aaggagatga cacaaaagtt agatctcatc 1740
acaagtgata tggcaggtta a 1761

<210> 4
<211> 586
<212> PRT
<213> homo sapiens

<400> 4
Met Gly Phe Gly Ala Gly Gln Arg Leu Arg Pro Val Pro Ala Pro Arg
1               5                   10                  15
Ser Ser Ala Glu Glu Ala Ala Arg Pro Gly Gln Leu Arg Leu Gly Ile
            20                  25                  30
Arg Arg Gly Glu Ala Glu Leu Ala Lys Leu Ala Pro Ser Gly Val Met
        35                  40                  45
Val Pro Gly Ala Arg Gly Gly Gly Ala Leu Ala Arg Ala Ala Gly Arg
    50                  55                  60
Gly Leu Leu Ala Leu Leu Leu Ala Val Ser Ala Pro Leu Arg Leu Gln
65                  70                  75                  80
Ala Glu Glu Leu Gly Asp Gly Cys Gly His Leu Val Thr Tyr Gln Asp
                85                  90                  95
Ser Gly Thr Met Thr Ser Lys Asn Tyr Pro Gly Thr Tyr Pro Asn His
            100                 105                 110
Thr Val Cys Glu Lys Thr Ile Thr Val Pro Lys Gly Lys Arg Leu Ile
        115                 120                 125
Leu Arg Leu Gly Asp Leu Asp Ile Glu Ser Gln Thr Cys Ala Ser Asp
    130                 135                 140
Tyr Leu Leu Phe Thr Ser Ser Ser Asp Gln Tyr Gly Pro Tyr Cys Gly
145                 150                 155                 160
Ser Met Thr Val Pro Lys Glu Leu Leu Leu Asn Thr Ser Glu Val Thr
                165                 170                 175
Val Arg Phe Glu Ser Gly Ser His Ile Ser Gly Arg Gly Phe Leu Leu
            180                 185                 190
Thr Tyr Ala Ser Ser Asp His Pro Asp Leu Ile Thr Cys Leu Glu Arg
        195                 200                 205
Ala Ser His Tyr Leu Lys Thr Glu Tyr Ser Lys Phe Cys Pro Ala Gly
    210                 215                 220
Cys Arg Asp Val Ala Gly Asp Ile Ser Gly Asn Met Val Asp Gly Tyr
225                 230                 235                 240
Arg Asp Thr Ser Leu Leu Cys Lys Ala Ala Ile His Ala Gly Ile Ile
                245                 250                 255
Ala Asp Glu Leu Gly Gly Gln Ile Ser Val Leu Gln Arg Lys Gly Ile
            260                 265                 270
Ser Arg Tyr Glu Gly Ile Leu Ala Asn Gly Val Leu Ser Arg Asp Gly
        275                 280                 285
Ser Leu Ser Asp Lys Arg Phe Leu Phe Thr Ser Asn Gly Cys Ser Arg

290                 295                 300
Ser Leu Ser Phe Glu Pro Asp Gly Gln Ile Arg Ala Ser Ser Ser Trp
305                 310                 315                 320
Gln Ser Val Asn Glu Ser Gly Asp Gln Val His Trp Ser Pro Gly Gln
                325                 330                 335
Ala Arg Leu Gln Asp Gln Gly Pro Ser Trp Ala Ser Gly Asp Ser Ser
            340                 345                 350
Asn Asn His Lys Pro Arg Glu Trp Leu Glu Ile Asp Leu Gly Glu Lys
        355                 360                 365
Lys Lys Ile Thr Gly Ile Arg Thr Thr Gly Ser Thr Gln Ser Asn Phe
    370                 375                 380
Asn Phe Tyr Val Lys Ser Phe Val Met Asn Phe Lys Asn Asn Asn Ser
385                 390                 395                 400
Lys Trp Lys Thr Tyr Lys Gly Ile Val Asn Asn Glu Glu Lys Val Phe
                405                 410                 415
Gln Gly Asn Ser Asn Phe Arg Asp Pro Val Gln Asn Asn Phe Ile Pro
            420                 425                 430
Pro Ile Val Ala Arg Tyr Val Arg Val Val Pro Gln Thr Trp His Gln
        435                 440                 445
Arg Ile Ala Leu Lys Val Glu Leu Ile Gly Cys Gln Ile Thr Gln Gly
    450                 455                 460
Asn Asp Ser Leu Val Trp Arg Lys Thr Ser Gln Ser Thr Ser Val Ser
465                 470                 475                 480
Thr Lys Lys Glu Asp Glu Thr Ile Thr Arg Pro Ile Pro Ser Glu Glu
                485                 490                 495
Thr Ser Thr Gly Ile Asn Ile Thr Thr Val Ala Ile Pro Leu Val Leu
            500                 505                 510
Leu Val Val Leu Val Phe Ala Gly Met Gly Ile Phe Ala Ala Phe Arg
        515                 520                 525
Lys Lys Lys Lys Lys Gly Ser Pro Tyr Gly Ser Ala Glu Ala Gln Lys
    530                 535                 540
Thr Asp Cys Trp Lys Gln Ile Lys Tyr Pro Phe Ala Arg His Gln Ser
545                 550                 555                 560
Ala Glu Phe Thr Ile Ser Tyr Asp Asn Glu Lys Glu Met Thr Gln Lys
                565                 570                 575
Leu Asp Leu Ile Thr Ser Asp Met Ala Gly
            580                 585

<210> 5
<211> 1620
<212> DNA
<213> homo sapiens

<400> 5
atggtgcccg gcgcccgcgg cggcggcgca ctggcgcggg ctgccgggcg gggcctcctg      60
gctttgctgc tcgcggtctc cgccccgctc cggctgcagg cggaggagct gggtgatggc     120
tgtggacacc tagtgactta tcaggatagt ggcacaatga catctaagaa ttatcccggg     180
acctacccca atcacactgt ttgcgaaaag acaattacag taccaaaggg gaaaagactg     240
attctgaggt tgggagattt ggatatcgaa tcccagacct gtgcttctga ctatcttctc     300
ttcaccagct cttcagatca atatggtcca tactgtggaa gtatgactgt tcccaaagaa     360
ctcttgttga acacaagtga agtaaccgtc cgctttgaga gtggatccca catttctggc     420
cggggttttt tgctgaccta tgcgagcagc gaccatccag atttaataac atgtttggaa     480
cgagctagcc attatttgaa gacagaatac agcaaattct gcccagctgg ttgtagagac     540
gtagcaggag acatttctgg gaatatggta gatggatata gagatacctc tttattgtgc     600
aaagctgcca tccatgcagg aataattgct gatgaactag gtggccagat cagtgtgctt     660
cagcgcaaag ggatcagtcg atatgaaggg attctggcca atggtgttct ttcgagggat     720
ggttccctgt cagacaagcg atttctgttt acctccaatg gttgcagcag atccttgagt     780
tttgaacctg acgggcaaat cagagcttct tcctcatggc agtcggtcaa tgagagtgga     840
gaccaagttc actggtctcc tggccaagcc cgacttcagg accaaggccc atcatgggct     900
tcgggcgaca gtagcaacaa ccacaaacca cgagagtggc tggagatcga tttgggggag     960
aaaaagaaaa taacaggaat taggaccaca ggatctacac agtcgaactt caacttttat    1020
gttaagagtt ttgtgatgaa cttcaaaaac aataattcta agtggaagac ctataaagga    1080
attgtgaata atgaagaaaa ggtgtttcag ggtaactcta actttcggga cccagtgcaa    1140
aacaatttca tccctcccat cgtggccaga tatgtgcggg ttgtccccca gacatggcac    1200
cagaggatag ccttgaaggt ggagctcatt ggttgccaga ttacacaagg taatgattca    1260
ttggtgtggc gcaagacaag tcaaagcacc agtgtttcaa ctaagaaaga agatgagaca    1320
atcacaaggc ccatcccctc ggaagaaaca tccacaggaa taaacattac aacggtggct    1380
attccattgg tgctccttgt tgtcctggtg tttgctggaa tggggatctt tgcagccttt    1440

agaaagaaga agaagaaagg aagtccgtat ggatcagcgg aggctcagaa aacagactgt 1500
tggaagcaga ttaaatatcc ctttgccaga catcagtcag ctgagtttac catcagctat 1560
gataatgaga aggagatgac acaaaagtta gatctcatca caagtgatat ggcaggttaa 1620

<210> 6
<211> 539
<212> PRT
<213> homo sapiens

<400> 6
Met Val Pro Gly Ala Arg Gly Gly Gly Ala Leu Ala Arg Ala Ala Gly
1               5                   10                  15
Arg Gly Leu Leu Ala Leu Leu Leu Ala Val Ser Ala Pro Leu Arg Leu
            20                  25                  30
Gln Ala Glu Glu Leu Gly Asp Gly Cys Gly His Leu Val Thr Tyr Gln
        35                  40                  45
Asp Ser Gly Thr Met Thr Ser Lys Asn Tyr Pro Gly Thr Tyr Pro Asn
    50                  55                  60
His Thr Val Cys Glu Lys Thr Ile Thr Val Pro Lys Gly Lys Arg Leu
65                  70                  75                  80
Ile Leu Arg Leu Gly Asp Leu Asp Ile Glu Ser Gln Thr Cys Ala Ser
                85                  90                  95
Asp Tyr Leu Leu Phe Thr Ser Ser Ser Asp Gln Tyr Gly Pro Tyr Cys
            100                 105                 110
Gly Ser Met Thr Val Pro Lys Glu Leu Leu Leu Asn Thr Ser Glu Val
        115                 120                 125
Thr Val Arg Phe Glu Ser Gly Ser His Ile Ser Gly Arg Gly Phe Leu
    130                 135                 140
Leu Thr Tyr Ala Ser Ser Asp His Pro Asp Leu Ile Thr Cys Leu Glu
145                 150                 155                 160
Arg Ala Ser His Tyr Leu Lys Thr Glu Tyr Ser Lys Phe Cys Pro Ala
                165                 170                 175
Gly Cys Arg Asp Val Ala Gly Asp Ile Ser Gly Asn Met Val Asp Gly
            180                 185                 190
Tyr Arg Asp Thr Ser Leu Leu Cys Lys Ala Ala Ile His Ala Gly Ile
        195                 200                 205
Ile Ala Asp Glu Leu Gly Gly Gln Ile Ser Val Leu Gln Arg Lys Gly
    210                 215                 220
Ile Ser Arg Tyr Glu Gly Ile Leu Ala Asn Gly Val Leu Ser Arg Asp
225                 230                 235                 240
Gly Ser Leu Ser Asp Lys Arg Phe Leu Phe Thr Ser Asn Gly Cys Ser
                245                 250                 255
Arg Ser Leu Ser Phe Glu Pro Asp Gly Gln Ile Arg Ala Ser Ser Ser
            260                 265                 270
Trp Gln Ser Val Asn Glu Ser Gly Asp Gln Val His Trp Ser Pro Gly
        275                 280                 285
Gln Ala Arg Leu Gln Asp Gln Gly Pro Ser Trp Ala Ser Gly Asp Ser
    290                 295                 300
Ser Asn Asn His Lys Pro Arg Glu Trp Leu Glu Ile Asp Leu Gly Glu
305                 310                 315                 320
Lys Lys Lys Ile Thr Gly Ile Arg Thr Thr Gly Ser Thr Gln Ser Asn
                325                 330                 335
Phe Asn Phe Tyr Val Lys Ser Phe Val Met Asn Phe Lys Asn Asn Asn
            340                 345                 350
Ser Lys Trp Lys Thr Tyr Lys Gly Ile Val Asn Asn Glu Glu Lys Val
        355                 360                 365
Phe Gln Gly Asn Ser Asn Phe Arg Asp Pro Val Gln Asn Asn Phe Ile
    370                 375                 380
Pro Pro Ile Val Ala Arg Tyr Val Arg Val Val Pro Gln Thr Trp His
385                 390                 395                 400
Gln Arg Ile Ala Leu Lys Val Glu Leu Ile Gly Cys Gln Ile Thr Gln
                405                 410                 415
Gly Asn Asp Ser Leu Val Trp Arg Lys Thr Ser Gln Ser Thr Ser Val
            420                 425                 430
Ser Thr Lys Lys Glu Asp Glu Thr Ile Thr Arg Pro Ile Pro Ser Glu
        435                 440                 445
Glu Thr Ser Thr Gly Ile Asn Ile Thr Thr Val Ala Ile Pro Leu Val
    450                 455                 460

Leu Leu Val Val Leu Val Phe Ala Gly Met Gly Ile Phe Ala Ala Phe
465                 470                 475                 480
Arg Lys Lys Lys Lys Lys Gly Ser Pro Tyr Gly Ser Ala Glu Ala Gln
                485                 490                 495
Lys Thr Asp Cys Trp Lys Gln Ile Lys Tyr Pro Phe Ala Arg His Gln
            500                 505                 510
Ser Ala Glu Phe Thr Ile Ser Tyr Asp Asn Glu Lys Glu Met Thr Gln
        515                 520                 525
Lys Leu Asp Leu Ile Thr Ser Asp Met Ala Gly
    530                 535

<210> 7
<211> 1768
<212> DNA
<213> homo sapiens

<400> 7
ggcggaggag ctgggtgatg gctgtggaca cctagtgact tatcaggata gtggcacaat      60
gacatctaag aattatcccg ggacctaccc caatcacact gtttgcgaaa agacaattac     120
agtaccaaag gggaaaagac tgattctgag gttgggagat ttggatatcg aatcccagac     180
ctgtgcttct gactatcttc tcttcaccag ctcttcagat caatatggtc catactgtgg     240
aagtatgact gttcccaaag aactcttgtt gaacacaagt gaagtaaccg tccgctttga     300
gagtggatcc cacatttctg gccggggttt tttgctgacc tatgcgagca gcgaccatcc     360
agatttaata acatgtttgg aacgagctag ccattatttg aagacagaat acagcaaatt     420
ctgcccagct ggttgtagag acgtagcagg agacatttct gggaatatgg tagatggata     480
tagagatacc tctttattgt gcaaagctgc catccatgca ggaataattg ctgatgaact     540
aggtggccag atcagtgtgc ttcagcgcaa agggatcagt cgatatgaag ggattctggc     600
caatggtgtt ctttcgaggg atggttccct gtcagacaag cgatttctgt ttacctccaa     660
tggttgcagc agatccttga gttttgaacc tgacgggcaa atcagagctt cttcctcatg     720
gcagtcggtc aatgagagtg gagaccaagt tcactggtct cctggccaag cccgacttca     780
ggaccaaggc ccatcatggg cttcgggcga cagtagcaac aaccacaaac cacgagagtg     840
gctggagatc gatttggggg agaaaaagaa aataacagga attaggacca caggatctac     900
acagtcgaac ttcaactttt atgttaagag ttttgtgatg aacttcaaaa acaataattc     960
taagtggaag acctataaag gaattgtgaa taatgaagaa aaggtgtttc agggtaactc    1020
taactttcgg gacccagtgc aaaacaattt catccctccc atcgtggcca gatatgtgcg    1080
ggttgtcccc cagacatggc accagaggat agccttgaag gtggagctca ttggttgcca    1140
gattacacaa ggtaatgatt cattggtgtg gcgcaagaca agtcaaagca ccagtgtttc    1200
aactaagaaa gaagatgaga caatcacaag gcccatcccc tcggaagaaa catccacagg    1260
aataaacatt acaacggtgg ctattccatt ggtgctcctt gttgtcctgg tgtttgctgg    1320
aatggggatc tttgcagcct ttagaaagaa gaagaagaaa ggaagtccgt atggatcagc    1380
ggaggctcag aaaacagact gttggaagca gattaaatat ccctttgcca gacatcagtc    1440
agctgagttt accatcagct atgataatga gaaggagatg acacaaaagt tagatctcat    1500
cacaagtgat atggcaggtt aactccgttg actgccaaaa tagcatcccc aacgtgcagc    1560
cctccgcatc tatcagcagg ttgccccgga tggatctcag agatgaggat tggaacacca    1620
tgttctttcc caccctaaca acaacaaagg gcagtaaatt aaagtactct ttgtaaggta    1680
cagttaccga ttaatctaga gataaaatat tttcttaaaa atatatttca ttaaacacct    1740
atgctgtctc tatgcaaaaa aaaaaaaa                                        1768

## Description

The present invention claims the benefit of U.S. Provisional Application Numbers 60/160,285 and 60/183,583 which were filed October 19, 1999 and February 18, 2000 respectively and are herein incorporated in their entirety.

### 1. INTRODUCTION

The present invention relates to the discovery, identification, and characterization of novel human polynucleotides encoding proteins sharing sequence similarity with mammalian proteins having CUB domains. The invention encompasses the described polynucleotides, host cell expression systems, the encoded proteins, fusion proteins, polypeptides and peptides, antibodies to the encoded proteins and peptides, and genetically engineered animals that either lack or over express the disclosed sequences, antagonists and agonists of the proteins, and other compounds that modulate the expression or activity of the proteins encoded by the disclosed polynucleotides that can be used for diagnosis, drug screening, clinical trial monitoring, or the treatment of physiological disorders, or diseases.

### 2. BACKGROUND OF THE INVENTION

The CUB domain is an extracellular domain (ECD) present in variety of diverse proteins such as bone morphogenetic protein 1, proteinases, spermadhesins, complement subcomponents, and neuronal recognition molecules. Given the importance of these functions, CUB proteins have been associated with, *inter alia,* regulating development, modulating cellular processes, and preventing infectious disease.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with animal CUB domain proteins, coagulation factors V and XIII, milk fat globule-EGF factor 8, transcriptional repressor AE-binding protein-1, and neuropilins 1 and 2 (which, like the presently described protein, contain both CUB and discoidin domains).

The novel human nucleic acid (cDNA) sequences described herein, encode proteins/open reading frames (ORFs) of 487, 586, and 539 amino acids in length (see SEQ ID NOS: 2, 4, and 6 respectively).

The invention also encompasses agonists and antagonists of the described NHPs, including small molecules, large molecules, mutant NHPs, or portions thereof that compete with native NHPs, NHP peptides, and antibodies, as well as nucleotide sequences that can be used to inhibit the expression of the described NHPs (*e.g*., antisense and ribozyme molecules, and gene or regulatory sequence replacement constructs) or to enhance the expression of the described NHPs (*e.g*., expression constructs that place the described gene under the control of a strong promoter system), and transgenic animals that express a NHP transgene, or "knock-outs" (which can be conditional) that do not express a functional NHP. Several knockout ES cell lines have been produced that contain a gene trap mutation in a murine ortholog/homolog of the disclosed NHPs.

Further, the present invention also relates to processes for identifying compounds that modulate, *i.e*., act as agonists or antagonists, of NHP expression and/or NHP activity that utilize purified preparations of the described NHPs and/or NHP products, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of several NHP ORFs encoding the described NHP amino acid sequences. SEQ ID NO:7 describes a NHP ORF and flanking sequences.

### 5. DETAILED DESCRIPTION OF THE INVENTION

The NHPs described for the first time herein are novel proteins that are expressed in, inter *alia,* human cell lines, and human prostate, pituitary, fetal brain, brain, thymus, spleen, lymph node, trachea, kidney, fetal liver, thyroid, adrenal gland, salivary gland, stomach, small intestine, colon, muscle, heart, mammary gland, adipose, skin, esophagus, bladder, cervix, rectum, and testis cells.

The described sequences were compiled from gene trapped cDNAs, genomic sequence, and clones isolated from human brain, adipose, testis, and placenta cDNA libraries (Edge Biosystems, Gaithersburg, MD, and Clontech, Palo Alto, CA). The present invention encompasses the nucleotides presented in the Sequence Listing, host cells expressing such nucleotides, the expression products of such nucleotides, and: (a) nucleotides that encode mammalian homologs of the described genes, including the specifically described NHPs, and NHP products; (b) nucleotides that encode one or more portions of a NHP that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (e.g., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF), or a contiguous exon splice junction first described in the Sequence Listing, that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et *al.*, eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of the DNA sequence that encode and express an amino acid sequence presented in the Sequence Listing under moderately stringent conditions, *e.g.,* washing in 0.2xSSC/0.1% SDS at 42°C (Ausubel et al., 1989, *supra),* yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding a NHP ORF, or its functional equivalent, encoded by a polynucleotide sequence that is about 99, 95, 90, or about 85 percent similar or identical to corresponding regions of the nucleotide sequences of the Sequence Listing (as measured by BLAST sequence comparison analysis using, for example, the GCG sequence analysis package using standard default settings).

The invention also includes nucleic acid molecules, preferably DNA molecules, that hybridize to, and are therefore the complements of, the described NHP gene nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. The oligonucleotides, typically between about 16 to about 40 (or any whole number within the stated range) nucleotides in length may partially overlap each other and/or a NHP sequence may be represented using oligonucleotides that do not overlap. Accordingly, the described NHP polynucleotide sequences shall typically comprise at least about two or three distinct oligonucleotide sequences of at least about 18, and preferably about 25, nucleotides in length that are each first disclosed in the described Sequence Listing. Such oligonucleotide sequences may begin at any nucleotide present within a sequence in the Sequence Listing and proceed in either a sense (5'-to-3') orientation vis-a-vis the described sequence or in an antisense orientation.

For oligonucleotide probes, highly stringent conditions may refer, *e.g*., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*:6625-6641). The oligonucleotide is a 2'-0-methylribonucleotide (Inoue *et al.,* 1987, Nucl. Acids Res.15:6131-6148), or a chimeric RNA-DNA analogue (Inoue et *al*., 1987, FEBS Lett. *215*:327-330). Alternatively, double stranded RNA can be used to disrupt the expression and function of a targeted NHP.

Oligonucleotides of the invention can be synthesized by standard methods known in the art, e.g. by use of an automated DNA synthesizer (such as are commercially available from Biosearch, Applied Biosystems, etc.). As examples, phosphorothioate oligonucleotides can be synthesized by the method of Stein *et al*. (1988, Nucl. Acids Res. *16*:3209), and methylphosphonate oligonucleotides can be prepared by use of controlled pore glass polymer supports (Sarin *et al*., 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook *et al*., 1989, Molecular Cloning, A Laboratory Manual (and periodic updates thereof), Cold Springs Harbor Press, N.Y.; and Ausubel *et al*., 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g*., splice acceptor and/or donor sites), etc., that can be used in diagnostics and pharmacogenomics.

Further, a NHP homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene. The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e*., one known, or suspected, to express a NHP gene, such as, for example, testis tissue). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see e.g., Sambrook *et al*., 1989, *supra.*

A cDNA encoding a mutant NHP gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of or known to carry a mutant NHP allele (*e*.*g*., a person manifesting a NHP-associated phenotype such as, for example, obesity, high blood pressure, connective tissue disorders, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against normal NHP product, as described below. (For screening techniques, see, for example, Harlow, E. and Lane, eds., 1988, "Antibodies: A Laboratory Manual", Cold Spring Harbor Press, Cold Spring Harbor.) Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e.g.*, as a result of a missense or a frameshift mutation), polyclonal antibodies to NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.*, antisense); (b) DNA expression vectors that contain any of the foregoing NHP coding sequences operatively associated with a regulatory element that directs the expression of the coding sequences (for example, baculo virus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i.e.*, gene activation). As used herein, regulatory elements include, but are not limited to, inducible and non-inducible promoters, enhancers, operators and other elements known to those skilled in the art that drive and regulate expression. Such regulatory elements include but are not limited to the human cytomegalovirus (hCMV) immediate early gene, regulatable, viral elements (particularly retroviral LTR promoters), the early or late promoters of SV40 adenovirus, the lac system, the trp system, the TAC system, the TRC system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of a NHP, as well as compounds or nucleotide constructs that inhibit expression of a NHP gene (transcription factor inhibitors, antisense and ribozyme molecules, or gene or regulatory sequence replacement constructs), or promote the expression of a NHP (e.g., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHPs or NHP peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for a NHP, but can also identify compounds that trigger NHP-mediated activities or pathways.

Finally, the NHP products can be used as therapeutics. For example, soluble derivatives such as NHP peptides/domains corresponding to NHP, NHP fusion protein products (especially NHP-Ig fusion proteins, *i.e.,* fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics a NHP could activate or effectively antagonize the endogenous NHP receptor. Nucleotide constructs encoding such NHP products can be used to genetically engineer host cells to express such products *in vivo;* these genetically engineered cells function as "bioreactors" in the body delivering a continuous supply of a NHP, a NHP peptide, or a NHP fusion protein to the body. Nucleotide constructs encoding functional NHP, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and corresponding deduced amino acid sequences of the described NHPs are presented in the Sequence Listing. SEQ ID NO:7 describes a NHP ORF as well as flanking regions. The NHP nucleotides were obtained from human cDNA libraries using probes and/or primers generated from human gene trapped sequence tags, and genomic sequence. Expression analysis has provided evidence that the described NHP can be expressed a variety of human cells as well as gene trapped human cells.

### 5.2 NHPs AND NHP POLYPEPTIDES

NHPs, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, for the identification of other cellular gene products related to a NHP, as reagents in assays for screening for compounds that can be as pharmaceutical reagents useful in the therapeutic treatment of mental, biological, or medical disorders and disease.

The Sequence Listing discloses the amino acid sequences encoded by the described NHP polynucleotides. The NHPs display an initiator methionines in DNA sequence contexts consistent with a translation initiation site, and several of the ORFs display a consensus signal sequence which can indicate that the described NHP ORFs are secreted proteins, or can be membrane associated.

The NHP amino acid sequences of the invention include the amino acid sequences presented in the Sequence Listing as well as analogues and derivatives thereof. Further, corresponding NHP homologues from other species are encompassed by the invention. In fact, any NHPs encoded by a NHP nucleotide sequence described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al*. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e.g*., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions can be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, a NHP peptide or NHP polypeptide is thought to be a soluble or secreted molecule, the peptide or polypeptide can be recovered from the culture media. Such expression systems also encompass engineered host cells that express NHP, or functional equivalent, *in situ.* Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of a NHP, but to assess biological activity, e.g., in drug screening assays.

The expression systems that may be used for purposes of the invention include but are not limited to microorganisms such as bacteria *(e.g., E. coli, B.* subtilis) transformed with recombinant bacteriophage DNA, plasmid DNA or cosmid DNA expression vectors containing NHP nucleotide sequences; yeast *(e.g., Saccharomyces, Pichia)* transformed with recombinant yeast expression vectors containing NHP encoding nucleotide sequences; insect cell systems infected with recombinant virus expression vectors (*e.g*., baculovirus) containing NHP sequences; plant cell systems infected with recombinant virus expression vectors (*e*.*g*., cauliflower mosaic virus, CaMV; tobacco mosaic virus, TMV) or transformed with recombinant plasmid expression vectors (*e.g*., Ti plasmid) containing NHP nucleotide sequences; or mammalian cell systems (e.g., COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of and/or containing a NHP, or for raising antibodies to a NHP, vectors that direct the expression of high levels of fusion protein products that are readily purified may be desirable. Such vectors include, but are not limited, to the *E. coli* expression vector pUR278 (Ruther *et al*., 1983, EMBO J. *2*:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109; Van Heeke & Schuster, 1989, J. Biol. Chem. 264:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP coding sequence can be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e.*, virus lacking the proteinaceous coat coded for by the polyhedrin gene). These recombinant viruses are then used to infect Spodoptera frugiperda cells in which the inserted sequence is expressed (*e.g*., see Smith *et al.*, 1983, J. Virol. 46: 584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, *e.g*., the late promoter and tripartite leader sequence. This chimeric gene may then be inserted in the adenovirus genome by in vitro or in vivo recombination. Insertion in a non-essential region of the viral genome (*e.g.*, region E1 or E3) will result in a recombinant virus that is viable and capable of expressing a NHP product in infected hosts (*e.g*., See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP sequence or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bittner et *al*., 1987, Methods in Enzymol. *153*:516-544).

In addition, a host cell strain may be chosen that modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express a NHP product. Such engineered cell lines may be particularly useful in screening and evaluation of compounds that affect the endogenous activity of a NHP product.

A number of selection systems may be used, including but not limited to the herpes simplex virus thymidine kinase (Wigler, *et al*., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al*., 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA 77:3567; O'Hare, *et al*., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et *al*., 1981, J. Mol. Biol. 150:1); and hygro, which confers resistance to hygromycin (Santerre, *et al*., 1984, Gene *30*:147).

Alternatively, any fusion protein can be readily purified by utilizing an antibody specific for the fusion protein being expressed. For example, a system described by Janknecht et *al*. allows for the ready purification of non-denatured fusion proteins expressed in human cell lines (Janknecht, et *al*., 1991, Proc. Natl. Acad. Sci. USA *88*:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of a NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP sequence product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with a NHP, an NHP peptide (*e.g.*, one corresponding to a functional domain of a NHP), truncated NHP polypeptides (NHP in which one or more domains have been deleted), functional equivalents of a NHP or mutated variants of a NHP. Such host animals may include but are not limited to pigs, rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum*. Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor *et al.,* 1983, Immunology Today *4*:72; Cole *et al*., 1983, Proc. Natl. Acad. Sci. USA 80:2026-2030), and the EBV-hybridoma technique (Cole *et al*., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison et al., 1984, Proc. Natl. Acad. Sci., *81*:6851-6855; Neuberger *et al.,* 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures which are herein incorporated by reference in their entirety.

Alternatively, techniques described for the production of single chain antibodies (U.S. Patent 4,946,778; Bird, 1988, Science *242*:423-426; Huston *et al*., 1988, Proc. Natl. Acad. Sci. USA *85*:5879-5883; and Ward *et al*., 1989, Nature *334*:544-546) can be adapted to produce single chain antibodies against NHP sequence products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al.*, 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, *e.g*., Greenspan & Bona, 1993, FASEB J 7(5):437-444; and Nissinoff, 1991, J. Immunol. *147(8)*:2429-2438). For example antibodies which bind to a NHP domain and competitively inhibit the binding of NHP to its cognate receptor can be used to generate anti-idiotypes that "mimic" a NHP and, therefore, bind and activate or neutralize a receptor. Such anti-idiotypic antibodies or Fab fragments of such anti-idiotypes can be used in therapeutic regimens involving a NHP signaling pathway.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. All cited publications, patents, and patent applications are herein incorporated by reference in their entirety.

## Claims

1. An isolated nucleic acid molecule comprising at least 24 contiguous bases of nucleotide sequence first disclosed in the NHP sequence described in SEQ ID NO: 3.

2. An isolated nucleic acid molecule comprising a nucleotide sequence that:
(a) encodes the amino acid sequence shown in SEQ ID NO: 4; and
(b) hybridizes under stringent conditions to the nucleotide sequence of SEQ ID NO: 3 or the complement thereof.

3. An isolated nucleic acid molecule according to claim 1 wherein said nucleotide sequence is present in cDNA.

4. An isolated nucleic acid molecule encoding the amino acid sequence presented in SEQ ID NO: 2.

5. An isolated nucleic acid molecule encoding the amino acid sequence presented in SEQ ID NO: 6.

6. An isolated polypeptide encoded by a nucleic acid as claimed in any one of claims 1 to 5.

7. An antibody that binds to a polypeptide as claimed in claim 6, or a fragment or a derivative of said antibody, for example, a polyclonal or monoclonal antibody, a single chain antibody, a Fab fragment, a F(ab')2, a fragment produced by a Fab expression library, or an anti-idiotypic (anti-Id) antibody, or an epitope binding fragment of any of said antibodies or fragments.

8. A host cell that comprises a nucleic acid as claimed in any one of claims 1 to 5 and is capable of expressing the polypeptide encoded by said nucleic acid, optionally on the surface of said host cell.

9. An animal that is capable of expressing a nucleic acid as claimed in any one of claims 1 to 5, or that is an optionally conditional "knock-out" animal that does not express a functional polypeptide as claimed in claim 6.

10. Use of a nucleic acid as claimed in any one of claims 1 to 5, a polypeptide as claimed in claim 6, an antibody as claimed in claim 7, a host cell as claimed in claim 8 or an animal as claimed in claim 9, in a method for screening compounds for the ability to modulate the expression of a nucleic acid as claimed in any one of claims 1 to 5 or a polypeptide as claimed in claim 6, or that modulates the activity of a polypeptide as claimed in claim 6, for example, for high throughput screening of combinatorial libraries.
